# EUROPÄISCHE PATENTANMELDUNG

(11) **EP 0 679 697 A2**
(43) Veröffentlichungstag der Anmeldung: **02.11.1995**
(21) Anmeldenummer: 95105627.4
(22) Anmeldetag: 13.04.1995
(51) Int. Cl.: C09B 67/22, C09B 67/26, D06P 1/384, D06P 1/38, C09B 62/507

(54) **Schwarze Farbstoffmischungen von faserreaktiven Azofarbstoffen und ihre Verwendung zum Färben von hydroxy- und/oder carbonamidgruppenhaltigem Fasermaterial**

(30) Priorität: 25.04.1994 DE 4414320
(71) Anmelder: HOECHST AKTIENGESELLSCHAFT, D-65929 Frankfurt am Main (DE)
(72) Erfinder: Dannheim, Jörg, Dr., D-60489 Frankfurt am Main (DE); Hohmann, Kurt, D-63263 Neu-Isenburg (DE); Russ, Werner Hubert, Dr., D-65439 Flörsheim (DE); Lippert, Gerhard, D-65719 Hofheim (DE)

(57) **Zusammenfassung**

Es werden Mischungen von faserreaktiven Azofarbstoffen beschrieben, mit denen schwarze Färbungen (einschließlich Drucke) auf hydroxy- und/oder carbonamidgruppenhaltigen Fasermaterialien, wie beispielsweise Cellulosefasermaterialien, Wolle und synthetische Polyamidfasern, erhalten werden. Diese Farbstoffmischungen enthalten eine oder mehrere Farbstoffe entsprechend den nachstehenden allgemeinen Formeln (1) und (2A) und/oder (2B)

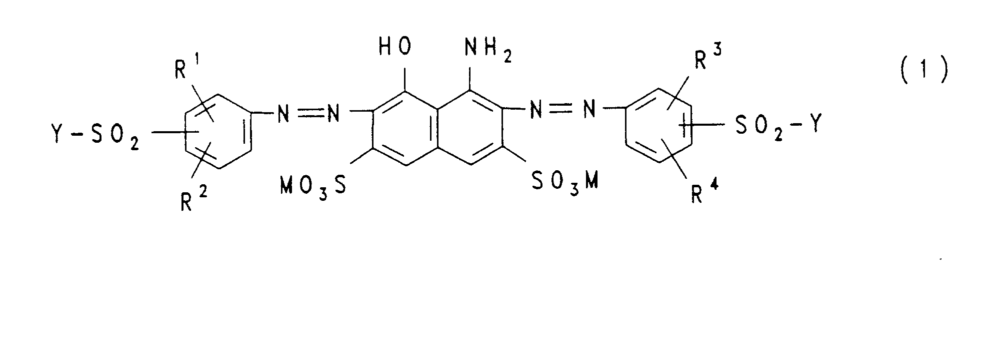

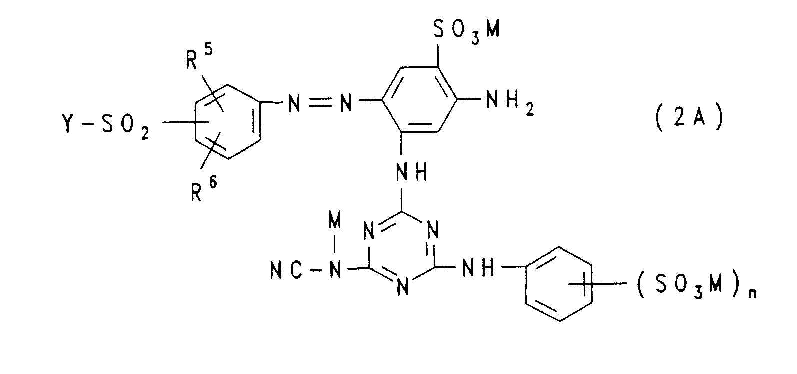

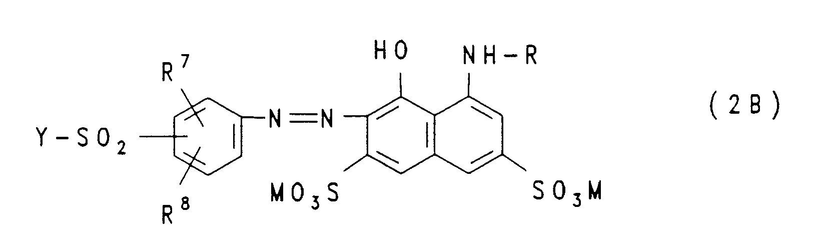

in welchen bedeuten: M ist Wasserstoff oder ein Alkalimetall, R¹ ist Wasserstoff, Methyl, Ethyl, Methoxy, Ethoxy, Sulfo oder Carboxy, R² ist Wasserstoff, Methyl, Ethyl, Methoxy oder Ethoxy, R³, R⁵ und R⁷ haben eine der Bedeutungen von R¹, R⁴, R⁶ und R⁸ haben eine der Bedeutungen von R², n ist die Zahl 1 oder 2, Y ist jedes Vinyl, β-Chlorethyl, β-Thiosulfatoethyl oder β-Sulfatoethyl, R ist niederes Alkanoyl, Benzoyl, 2,4-Di-(Cyanoamino)-1,3,5-triazin-6-yl oder eine Gruppe der Formeln (3a) oder (3b) 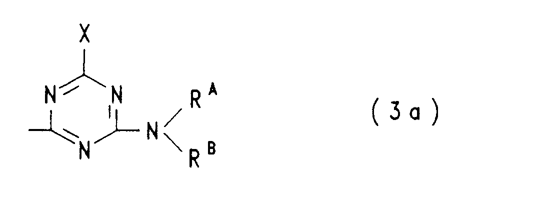 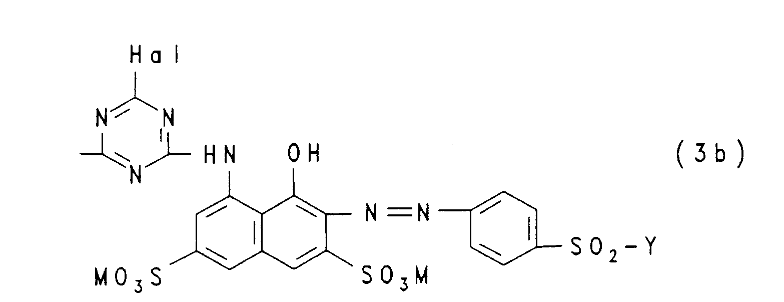
in welchen M und Y die genannten Bedeutungen haben, X Chlor, Fluor oder Cyanoamino ist, Hal Chlor oder Fluor ist, R^{A} Wasserstoff, niederes Alkyl oder gegebenenfalls substituiertes Phenyl ist, R^{B} Mono- oder Disulfo-phenyl ist oder R^{A} und R^{B} zusammen mit dem Stickstoffatom einen gesättigten Heterocyclus bilden.

## Beschreibung

Die Erfindung liegt auf dem technischen Gebiet der faserreaktiven Farbstoffe.

Schwarzfärbende Farbstoffmischungen sind bereits aus den japanischen Patentanmeldungs-Veröffentlichungen Hei-2-073870 und Hei-2-202956 sowie aus den koreanischen Patentschriften Nrs. 91/2676, 91/6386 und 91/8343 bekannt, die sich zur Herstellung von tiefschwarzen Färbungen auf Cellulosefasermaterialien eignen. Diese bekannten Farbstoffmischungen weisen jedoch gewisse anwendungstechnische Mängel auf; so ist insbesondere ihre Auswaschbarkeit und ihre Naßliegeechtheit verbesserungsbedürftig.

Mit der vorliegenden Erfindung wurden Farbstoffmischungen gefunden, die einen oder mehrere, wie 2, 3 oder 4, Disazofarbstoffe entsprechend der allgemeinen Formel (1) und einen oder mehrere, wie 2, 3 oder 4, Mono- oder Disazofarbstoffe entsprechend der allgemeinen Formel (2A) oder der allgemeinen Formel (2B) oder eine Mischung dieser Azofarbstoffe der Formeln (2) enthalten. 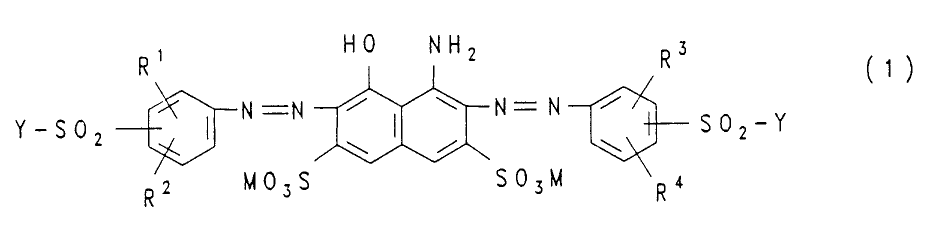 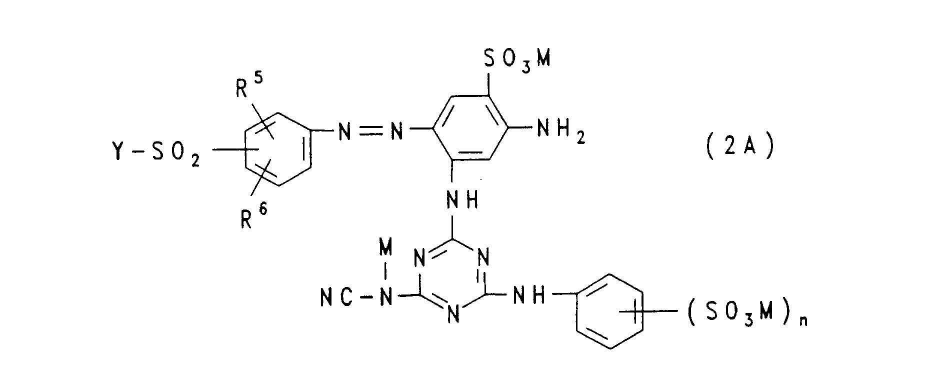 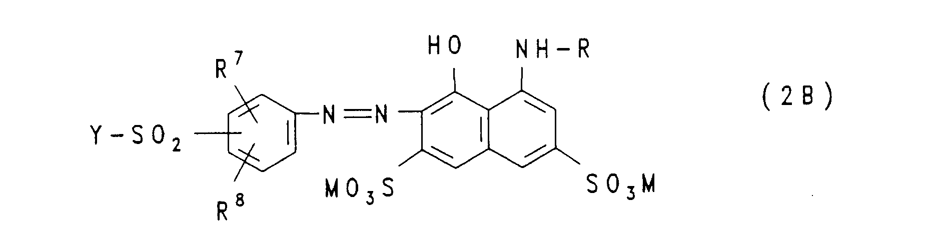
In diesen Formeln bedeuten
M ist Wasserstoff oder ein Alkalimetall, wie Lithium, Natrium und Kalium;
R¹ ist Wasserstoff, Methyl, Ethyl, Methoxy, Ethoxy, Sulfo oder Carboxy, vorzugsweise Methoxy und Wasserstoff und insbesondere Wasserstoff;
R² ist Wasserstoff, Methyl, Ethyl, Methoxy oder Ethoxy, vorzugsweise Wasserstoff;
R³ ist Wasserstoff, Methyl, Ethyl, Methoxy, Ethoxy, Sulfo oder Carboxy, vorzugsweise Methoxy und Wasserstoff und insbesondere Wasserstoff;
R⁴ ist Wasserstoff, Methyl, Ethyl, Methoxy oder Ethoxy, vorzugsweise Wasserstoff;
R⁵ ist Wasserstoff, Methyl, Ethyl, Methoxy, Ethoxy, Sulfo oder Carboxy, vorzugsweise Methoxy und Wasserstoff und insbesondere Wasserstoff;
R⁶ ist Wasserstoff, Methyl, Ethyl, Methoxy oder Ethoxy, vorzugsweise Wasserstoff;
R⁷ ist Wasserstoff, Methyl, Ethyl, Methoxy, Sulfo oder Carboxy, vorzugsweise Methoxy und Wasserstoff und insbesondere Wasserstoff;
R⁸ ist Wasserstoff, Methyl, Ethyl, Methoxy oder Ethoxy, vorzugsweise Wasserstoff;
n ist die Zahl 1 oder 2, bevorzugt 1;
Y ist jedes, unabhängiges voneinander, Vinyl, ß-Chlorethyl, β-Thiosulfatoethyl oder ß-Sulfatoethyl;
R ist Alkanoyl von 2 bis 5 C-Atomen, wie Propionyl und Acetyl, oder ist Benzoyl oder ist 2,4-Di-(cyanoamino)-1,3,5-triazin-6-yl oder ist eine Gruppe der allgemeinen Formel (3a) oder (3b) 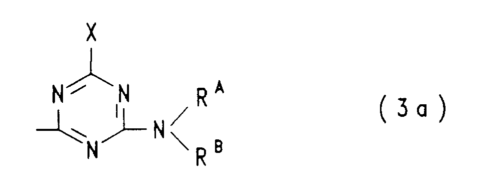 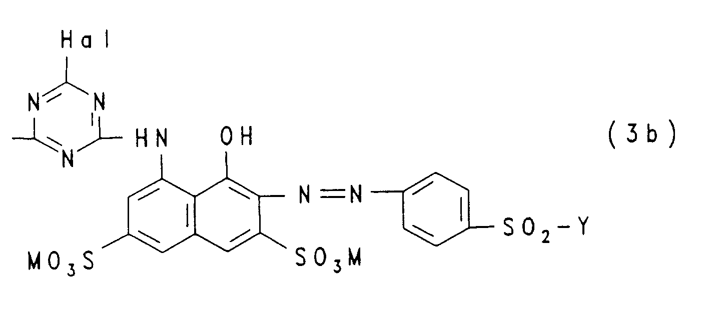
   in welchen
M und Y eine der obengenannten Bedeutungen haben,
X für Chlor, Fluor oder Cyanoamino steht,
Hal Chlor oder Fluor ist,
R^{A} Wasserstoff oder Alkyl von 1 bis 4 C-Atomen, wie Methyl oder Ethyl, oder Phenyl ist, das substituiert sein kann, wie beispielsweise durch 1 oder 2 Substituenten aus der Gruppe Sulfo, Carboxy, Methyl, Ethyl, Methoxy und Ethoxy, und bevorzugt Wasserstoff ist, und
R^{B} Monosulfo-phenyl oder Disulfo-phenyl ist, bevorzugt 3-Sulfo-phenyl ist, oder
R^{A} und R^{B} zusammen mit dem N-Atom einen gesättigten heterocyclischen Rest mit einem Alkylen von 4 bis 7 C-Atomen, bevorzugt von 5 C-Atomen, oder mit zwei Alkylenresten von 1 bis 4 C-Atomen und einer weiteren Heterogruppe, wie -O- oder -NH- , bilden, wie beispielsweise den N-Piperidino-, N-Piperazino- oder bevorzugt N-Morpholino-Rest; die Gruppen -S0₂-Y stehen bevorzugt in meta-Stellung und insbesondere bevorzugt in para-Stellung zur Azogruppe an den Benzolkern gebunden.

Die einzelnen Formelglieder in den Formeln (1) und (2) und ebenso in den nachfolgend genannten allgemeinen Formeln können im Rahmen ihrer Definition zueinander gleiche oder voneinander verschiedene Bedeutungen besitzen.

Bevorzugt ist in den Farbstoffen der allgemeinen Formel (2B) der Rest R gleich eine Gruppe der allgemeinen Formel (3a), in welcher X Chlor oder Fluor bedeutet.

Im allgemeinen sind der oder die Disazofarbstoffe der allgemeinen Formel (1) und der oder die Azofarbstoffe der allgemeinen Formel (2A) oder (2B) oder (2A) und (2B) in der Mischung in einem molaren Mischungsverhältnis von 1:0,3 bis 1:1,25, bevorzugt im Molverhältnis von (1):(2) von 1:0,4 bis 1:0,8, enthalten. Sind in den erfindungsgemäßen Farbstoffmischungen sowohl ein oder mehrere Farbstoffe der allgemeinen Formel (2A) als auch ein oder mehrere Farbstoffe der allgemeinen Formel (2B) enthalten, so liegen die Farbstoffe (2A) und (2B) bevorzugt in einem molaren Mischungsverhältnis von 1:0,2 bis 1:1 vor.

Reste von Diazokomponenten in den allgemeinen Formeln (1), (2A) und (2B) sind beispielsweise 3-(ß-Sulfatoethylsulfonyl)-phenyl, 4-(ß-Sulfatoethylsulfonyl)-phenyl, 2-Methyl-5-methoxy-4-(ß-sulfatoethylsulfonyl)-phenyl, 2-Methoxy-5-(ß-sulfatoethylsulfonyl)-phenyl und 2,5-Dimethoxy-4-(ß-sulfatoethylsulfonyl)-phenyl sowie deren Vinylsulfonyl-, β-Chlorethylsulfonyl- und β-Thiosulfatoethylsulfonyl-Derivate; hiervon bevorzugt sind 3-(ß-Sulfatoethylsulfonyl)-phenyl, 4-(ß-Sulfatoethylsulfonyl)-phenyl, 2-Methoxy-5-(β-sulfatoethylsulfo- nyl)-phenyl und 2,5-Dimethoxy-4-(β-sulfatoethylsulfonyl)-phenyl.

Die erfindungsgemäßen Farbstoffmischungen können zusätzlich 1 bis 15 Gew.-%, insbesondere 3 bis 10 Gew.-%, bezogen auf die erfindungsgemäße Farbstoffmischung, an einem oder mehreren, wie 2, 3 oder 4, Monoazofarbstoffen entsprechend den allgemeinen Formeln (2a) und/oder (2b) 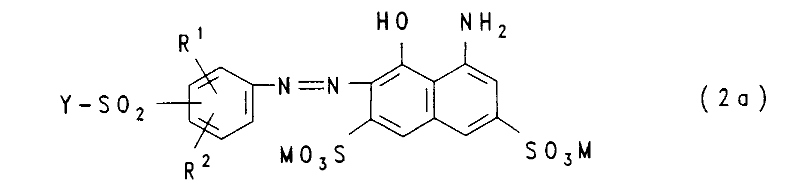 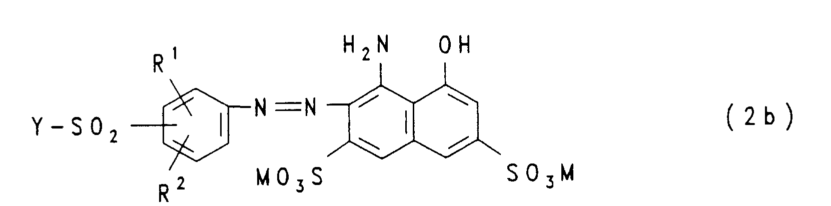
enthalten, in welchen R¹, R², M und Y eine der obengenannten Bedeutungen haben.

Des weiteren können die erfindungsgemäßen Mischungen noch mit einem gelben Monoazofarbstoff von bis zu 10 Gew.-%, vorzugsweise bis zu 5 Gew.-%, nuanciert sein. Solche bekannten Farbstoffe sind beispielsweise Monoazofarbstoffe der allgemeinen Formeln (a), (b) und (c) 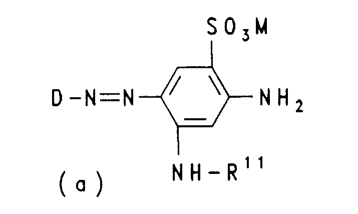 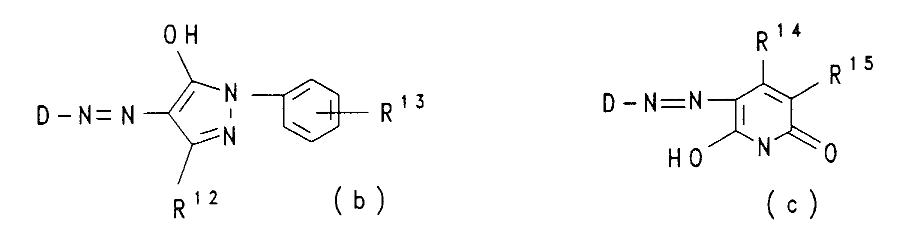
in welchen bedeuten:
D ist 3-(ß-Sulfatoethylsulfonyl)-phenyl, 4-(ß-Sulfatoethylsulfonyl)-phenyl, 3-Vinylsulfonyl-phenyl, 4-Vinylsulfonyl-phenyl, 2-Methoxy-5-(β-sulfatoethylsulfonyl)-phenyl oder 2-Methoxy-5-vinylsulfonyl- phenyl;
M hat eine der obengenannten Bedeutungen;
R¹¹ ist Acetyl, Carbamoyl oder 4,6-Di-cyanamido-1,3,5-triazin-2-yl, bevorzugt Acetyl,
R¹² ist Methyl oder Carboxy;
R¹³ ist Sulfo, β-Chlorethylsulfonyl oder β-Sulfatoethylsulfonyl;
R¹⁴ ist Wasserstoff oder Methyl;
R¹⁵ ist Wasserstoff, Cyano, Carbamoyl, Carboxy oder Sulfomethyl;
R¹⁶ ist Methyl, Ethyl oder ß-Sulfoethyl.

Die Farbstoffe der allgemeinen Formeln (1) und (2) sind zahlreich in der Literatur beschrieben. So sind die Disazofarbstoffe der allgemeinen Formel (1) beispielsweise aus der U.S.-Patentschrift 2 657 205, aus der japanischen Patentanmeldungs-Veröffentlichung Sho-58-160 362 und aus der U.S.-Patentschrift 4 257 770 und der dort genannten Literatur bekannt. Monoazo- und Disazofarbstoffe der allgemeinen Formeln (2) sind beispielsweise in den europäischen Patentanmeldungs-Veröffentlichungen Nrs. 0 040 806 und 0 542 214 und in der deutschen Patentschrift Nr. 2 748 965 beschrieben oder können analog den dort gemachten Angaben synthetisiert werden.

Eine Sulfogruppe ist eine Gruppe der allgemeinen Formel -SO₃M , eine Carboxygruppe ist eine Gruppe der allgemeinen Formel -COOM , eine Sulfatogruppe ist eine Gruppe der allgemeinen Formel -OSO₃M und eine Thiosulfatogruppe ist eine Gruppe der allgemeinen Formel -S-SO₃M .

Die Farbstoffe der Formel (1) und ebenso die Farbstoffe der Formeln (2) können, insbesondere bei gleichem Chromophor, innerhalb der Bedeutung von Y unterschiedliche faserreaktive Gruppen -S0₂-Y besitzen. Insbesondere können die Farbstoffmischungen Farbstoffe gleichen Chromophors entsprechend der allgemeinen Formel (1) und/oder Farbstoffe gleichen Chromophors entsprechend den allgemeinen Formeln (2) enthalten, in denen die faserreaktiven Gruppen -S0₂-Y zum einen Vinylsulfonylgruppen und zum anderen β-Chlorethylsulfonyl- oder β-Thiosulfatoethylsulfonyl- oder bevorzugt β-Sulfatoethylsulfonyl-Gruppen sind. Ist in den Farbstoffgemischen solch eine Farbstoffkomponente in Form eines VinylsulfonylFarbstoffes vorhanden, so liegt der Farbstoffanteil dieses Vinylsulfonylfarbstoffes zu dem entsprechenden ß-Chlor- oder ß-Thiosulfato- oder β-Sulfatoethylsulfonyl-Farbstoff bei bis zu etwa 30 Mol-%, bezogen auf den Farbstoffchromophor, vor.

Hierbei sind solche Farbstoffmischungen bevorzugt, bei welchen der Anteil an den Vinylsulfonyl-Farbstoffen zu den β-Sulfatoethylsulfonyl-Farbstoffen im Molverhältnis zwischen 2:98 und 30:70 liegt.

Bevorzugt sind in den erfindungsgemäßen Farbstoffmischungen Farbstoffe der allgemeinen Formel (1) solche, die der nachstehend angegebenen allgemeinen Formel (10) entsprechen, und Farbstoffe der allgemeinen Formel (2A) solche, die der nachstehend angegebenen und definierten allgemeinen Formel (11A) entsprechen, und Farbstoffe der allgemeinen Formel (2B) solche, die der nachstehend angegebenen und definierten allgemeinen Formel (11 B) entsprechen 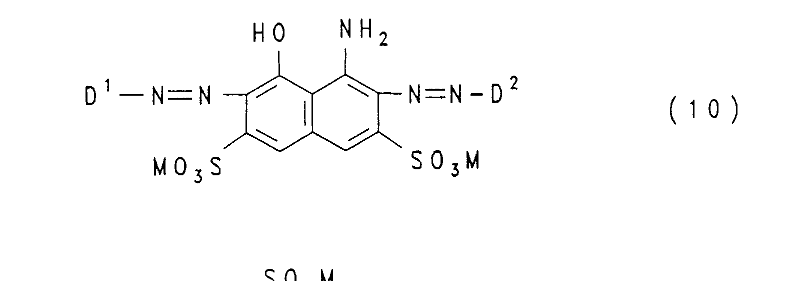 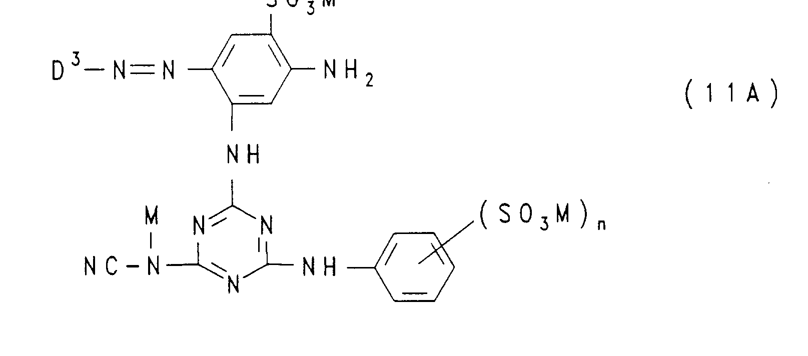 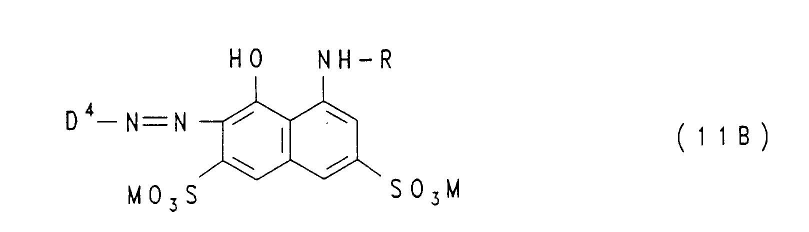
in welchen M, n und R eine der obengenannten Bedeutungen haben und D¹, D², D³ und D⁴ jedes, unabhängig voneinander, 3-Vinylsulfonyl-phenyl, 4-Vinylsulfonyl-phenyl, 3-(ß-Sulfatoethylsulfonyl)-phenyl oder 4-(ß-Sulfatoethylsulfonyl)-phenyl bedeutet, wobei im Falle, daß sowohl Vinylsulfonylals auch β-Sulfatoethylsulfonyl-Gruppen in den Farbstoffmischungen vorliegen, das molare Verhältnis zwischen den Vinylsulfonyl-Anteilen und den β-Sulfatoethylsulfonyl-Anteilen zwischen 2:98 und 30:70 liegt.

Weiterhin sind insbesondere Farbstoffmischungen aus einem oder mehreren Farbstoffen der allgemeinen Formel (10) und einem oder mehreren Farbstoffen der allgemeinen Formeln (11A) und/oder (11B) bevorzugt, in welchen D¹, D², D³ und D⁴, unabhängig voneinander, 3-(ß-Sulfatoethylsulfonyl)-phenyl, 4-(ß-Sulfatoethylsulfonyl)-phenyl, 3-Vinylsulfonyl-phenyl oder 4-Vinylsulfonyl-phenyl, bevorzugt 4-(ß-Sulfatoethyl- sulfonyl)-phenyl und/oder 4-Vinylsulfonylphenyl bedeuten, wobei im Falle, daß sowohl Vinylsulfonyl- als auch β-Sulfatoethylsulfonyl-Gruppen in den Farbstoffmischungen vorliegen, das molare Verhältnis zwischen den Vinylsulfonyl-Anteilen und den β-Sulfatoethylsulfonyl-Anteilen zwischen 2:98 und 30:70 liegt.

Die erfindungsgemäßen Farbstoffmischungen können als Präparation in fester oder in flüssiger (gelöster) Form vorliegen. Sie enthalten im allgemeinen die bei wasserlöslichen und insbesondere faserreaktiven Farbstoffen üblichen Elektrolytsalze, wie Natriumchlorid, Kaliumchlorid und Natriumsulfat, und können desweiteren die in Handelsfarbstoffen üblichen Hilfsmittel enthalten, wie Puffersubstanzen, die einen pH-Wert in wäßriger Lösung zwischen 3 und 7 einzustellen vermögen, wie Natriumacetat, Natriumborat, Natriumhydrogencarbonat, Natriumdihydrogenphosphat und Dinatriumhydrogenphosphat, bei Vorliegen in fester Form geringe Mengen an Sikkativen und in flüssiger, wäßriger Lösung (einschließlich des Gehaltes von Verdickungsmitteln, wie sie bei Druckpasten üblich sind) Substanzen, die die Haltbarkeit dieser Präparationen gewährleisten, wie beispielsweise schimmelverhütende Mittel.

Im allgemeinen liegen die erfindungsgemäßen Farbstoffmischungen als Farbstoffpulver mit einem Gehalt von 10 bis 80 Gew.-%, bezogen auf das Farbstoffpulver bzw, die Präparation, an einem Elektrolytsalz, das auch als Stellmittel bezeichnet wird, vor. Diese Farbstoffpulver können zudem die erwähnten Puffersubstanzen in einer Gesamtmenge von bis zu 5 %, bezogen auf das Farbstoffpulver,enthalten. Sofern die erfindungsgemäßen Farbstoffmischungen in wäßriger Lösung vorliegen, so beträgt der Gesamtfarbstoffgehalt in diesen wäßrigen Lösungen bis zu etwa 50 Gew.-%, wie beispielsweise zwischen 5 und 50 Gew.- %., wobei der Elektrolytsalzgehalt in diesen wäßrigen Lösungen bevorzugt unterhalb 10 Gew.-%, bezogen auf die wäßrige Lösung, beträgt; die wäßrigen Lösungen (Flüssigpräparationen) können die erwähnten Puffersubstanzen in der Regel in einer Menge von bis zu 5 Gew.-%, bevorzugt bis zu 2 Gew.-%, enthalten.

Die erfindungsgemäßen Farbstoffmischungen können in üblicher Weise hergestellt werden, so durch mechanisches Mischen der einzelnen Farbstoffe in den erforderlichen Anteilen oder durch Synthese mittels der üblichen Diazotierungs- und Kupplungsreaktionen unter Verwendung von entsprechenden Mischungen der Diazo- und Kupplungskomponenten in einer dem Fachmann geläufigen Weise und den hierzu erforderlichen Mengenanteilen. So kann man beispielsweise in der Weise vorgehen, daß man 1-Amino-8-naphthol-3,6-disulfonsäure mit einem Überschuß eines Diazoniumsalzes des Amins der allgemeinen Formel (4) 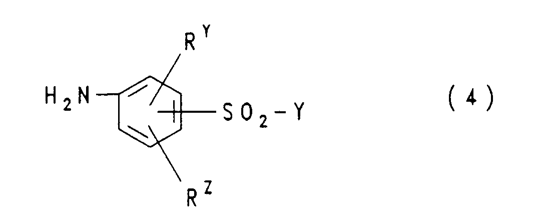
in welcher Y die obengenannte Bedeutung besitzt, R^{y} eine der Bedeutungen von R¹, R³, R⁵ und R⁷ und R^{z} eine der Bedeutungen von R², R⁴, R⁶ und R⁸ hat, zur Monoazoverbindung der allgemeinen Formel (5) 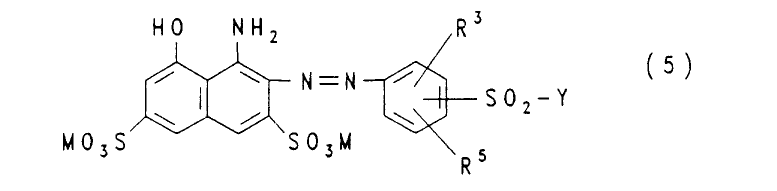
mit M, R³, R⁴ und Y der obengenannten Bedeutung bei einem pH-Wert von 0,3 bis 2,5 und einer Temperatur von 0 bis 20 °C umsetzt, danach den pH-Wert auf 4 bis 7 erhöht, zu dem Ansatz eine Verbindung der allgemeinen Formel (6) und/oder (7) 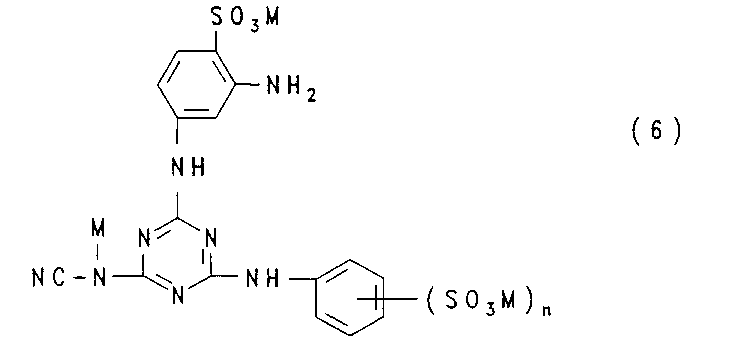 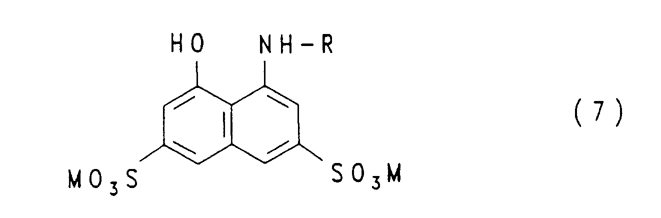
mit M, n und R der obengenannten Bedeutung zugibt und die Kupplungsreaktion zu den Azofarbstoffen der allgemeinen Formeln (1) und (2A) und/oder (2B) bei einem pH-Wert zwischen 4 und 7, bevorzugt zwischen 5 und 6, und einer Temperatur zwischen 0 und 20 _{°} C zu Ende führt.

Die so erhaltene Farbstoffmischung kann aus der Lösung in üblicher Weise isoliert werden, so beispielsweise durch Aussalzen mit einem Elektrolytsalz, wie Natriumchlorid, Kaliumchlorid oder Lithiumchlorid, oder durch Sprühtrocknung.

Werden die erfindungsgemäßen Farbstoffmischungen durch mechanisches Mischen der Einzelfarbstoffe hergestellt, so werden beim Mischen eventuell erforderliche Stellmittel, Entstaubungsmittel oder weitere Hilfsmittel, die in der Färbereitechnik oder in den hierzu verwendeten Farbstoffpräparationen üblich sind, zugegeben.

Die erfindungsgemäßen Farbstoffmischungen aus den Farbstoffen der Formeln (1) und (2) liefern nach den in der Technik für faserreaktive Farbstoffe zahlreich beschriebenen Anwendungs- und Fixierverfahren auf hydroxy- und/oder carbonamidgruppenhaltigen Fasermaterialien tiefschwarze Färbungen mit gutem Farbaufbau und guter Auswaschbarkeit nicht fixierter Farbstoffanteile.

Gegenstand der vorliegenden Erfindung ist somit auch die Verwendung der erfindungsgemäßen Farbstoffmischungen oder der Farbstoffe (1) und (2) gemeinsam zum Färben (einschließlich Bedrucken) von hydroxy- und/oder carbonamidgruppenhaltigen Fasermaterialien bzw. Verfahren zum Färben solcher Fasermaterialien unter Verwendung einer erfindungsgemäßen Farbstoffmischung oder der Farbstoffe (1) und (2) gemeinsam, indem man die Farbstoffmischung bzw. die Farbstoffe in gelöster Form auf das Substrat appliziert und die Farbstoffe durch Einwirkung eines alkalisch wirkenden Agens oder durch Hitze oder durch beide Maßnahmen auf der Faser fixiert.

Hydroxygruppenhaltige Materialien sind natürliche oder synthetische hydroxygruppenhaltige Materialien, wie beispielsweise Cellulosefasermaterialien, auch in Form von Papier, oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form der Fasern, beispielsweise Wolle und andere Tierhaare, Seide, Leder, Polyamid-6,6, Polyamid-6, Polyamid-11 und Polyamid-4.

Die Anwendung der erfindungsgemäßen Farbstoffmischungen erfolgt nach allgemein bekannten Verfahren zum Färben und Bedrucken von Fasermaterialien gemäß den bekannten Anwendungstechniken für faserreaktive Farbstoffe. Da die Farbstoffe der erfindungsgemäßen Farbstoffmischungen zueinander ein sehr gutes Kombinationsverhalten zeigen, können die erfindungsgemäßen Farbstoffmischungen auch mit Vorteil in den Ausziehfärbeverfahren eingesetzt werden. Demgemäß erhält man mit ihnen beispielsweise auf Cellulosefasern nach den Ausziehverfahren aus langer Flotte bei Temperaturen zwischen 40 und 1050 C, gegebenenfalls bei Temperaturen bis zu 130°C unter Druck, und gegebenenfalls in Gegenwart von üblichen Färbereihilfsmitteln unter Verwendung von säurebindenden Mitteln und gegebenenfalls neutralen Salzen, wie Natriumchlorid oder Natriumsulfat, Färbungen in sehr guten Farbausbeuten und mit ausgezeichnetem Farbaufbau und gleicher Nuance. Man kann dabei so vorgehen, daß man das Material in das warme Bad einbringt und dieses allmählich auf die gewünschte Färbetemperatur erwärmt und den Färbeprozeß bei dieser Temperatur zu Ende führt. Die das Ausziehen der Farbstoffe beschleunigenden Neutralsalze können dem Bade gewünschtenfalls auch erst nach Erreichen der eigentlichen Färbetemperatur zugesetzt werden.

Ebenfalls erhält man nach den üblichen Druckverfahren für Cellulosefasern - die entweder einphasig durchgeführt werden können, beispielsweise durch Bedrucken mit einer Natriumbicarbonat oder ein anderes säurebindendes Mittel und das Farbmittel enthaltenden Druckpaste und durch anschließendes Dämpfen bei 100 bis 103_{°}C, oder die zweiphasig, beispielsweise durch Bedrucken mit neutraler oder schwach saurer, das Farbmittel enthaltender Druckpaste und anschließendes Fixieren entweder durch Hindurchführen der bedruckten Ware durch ein heißes elektrolythaltiges alkalisches Bad oder durch Überklotzen mit einer alkalischen elektrolythaltigen Klotzflotte mit anschließendem Verweilen dieses behandelten Materials oder anschließendem Dämpfen oder anschließender Behandlung mit Trockenhitze, durchgeführt werden können, - farbstarke Drucke mit gutem Stand der Konturen und einem klaren Weißfond. Der Ausfall der Drucke ist von wechselnden Fixierbedingungen nur wenig abhängig. Sowohl in der Färberei als auch in der Druckerei sind die mit den erfindungsgemäßen Farbstoffmischungen erhaltenen Fixiergrade sehr hoch. Bei der Fixierung mittels Trockenhitze nach den üblichen Thermofixierverfahren verwendet man Heißluft von 120 bis 200 _{°} C. Neben dem üblichen Wasserdampf von 101 bis 1030 C kann auch überhitzter Dampf und Druckdampf von Temperaturen bis zu 160°C eingesetzt werden.

Die säurebindenden und die Fixierung der Farbstoffe auf den Cellulosefasern bewirkenden Mittel sind beispielsweise wasserlösliche basische Salze der Alkalimetalle und der Erdalkalimetalle von anorganischen oder organischen Säuren, ebenso Verbindungen, die in der Hitze Alkali freisetzen. Insbesondere sind die Alkalimetallhydroxide und Alkalimetallsalze von schwachen bis mittelstarken anorganischen oder organischen Säuren zu nennen, wobei von den Alkaliverbindungen vorzugsweise die Natrium- und Kaliumverbindungen gemeint sind. Solche säurebindenden Mittel sind beispielsweise Natriumhydroxid, Kaliumhydroxid, Natriumcarbonat, Natriumbicarbonat, Kaliumcarbonat, Natriumformiat, Natriumdihydrogenphosphat und Dinatriumhydrogenphosphat.

Durch die Behandlung der Farbstoffe der erfindungsgemäßen Farbstoffmischungen mit den säurebindenden Mitteln, gegebenenfalls unter Wärmeeinwirkung, werden die Farbstoffe chemisch an die Cellulosefaser gebunden; insbesondere die Cellulosefärbungen zeigen nach der üblichen Nachbehandlung durch Spülen zur Entfernung von nicht fixierten Farbstoffanteilen ausgezeichnete Naßechtheiten, zumal sich nicht fixierte Farbstoffanteile leicht wegen ihrer guten Kaltwasserlöslichkeit auswaschen lassen.

Die Färbungen auf Polyurethan- und Polyamidfasern werden üblicherweise aus saurem Milieu ausgeführt. So kann man beispielsweise dem Färbebad Essigsäure und/oder Ammoniumsulfat und/oder Essigsäure und Ammoniumacetat oder Natriumacetat zufügen, um den gewünschten pH-Wert zu erhalten. Zur Erreichung einer brauchbaren Egalität der Färbung empfiehlt sich ein Zusatz an üblichen Egalisierhilfsmitteln, wie beispielsweise auf Basis eines Umsetzungsproduktes von Cyanurchlorid mit der dreifach molaren Menge einer Aminobenzolsulfonsäure oder Aminonaphthalinsulfonsäure oder auf Basis eines Umsetzungsproduktes von beispielsweise Stearylamin mit Ethylenoxid. In der Regel wird das zu färbende Material bei einer Temperatur von etwa 40 _{°} C in das Bad eingebracht, dort einige Zeit darin bewegt, das Färbebad dann auf den gewünschten schwach sauren, vorzugsweise schwach essigsauren pH-Wert nachgestellt und die eigentliche Färbung bei einer Temperatur zwischen 60 und 98 _{°} C durchgeführt. Die Färbungen können aber auch bei Siedetemperatur oder bei Temperaturen bis zu 120°C (unter Druck) ausgeführt werden.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile stehen zu Volumenteilen im Verhältnis von Kilogramm zu Liter.

In den Beispielen sind die Formeln der Farbstoffe in Form der freien Säure angegeben; die Mengenteile beziehen sich auf die saure Form. In der Regel werden die Farbstoffe jedoch in der für wasserlösliche Farbstoffe üblicherweise vorliegenden Form als elektrolytsalzhaltiges (beispielsweise natriumchlorid- und natriumsulfathaltiges) Alkalimetallsalz-Pulver eingesetzt.

### Beispiel 1

24,8 Teile des marineblaufärbenden Disazofarbstoffes der Formel (A)

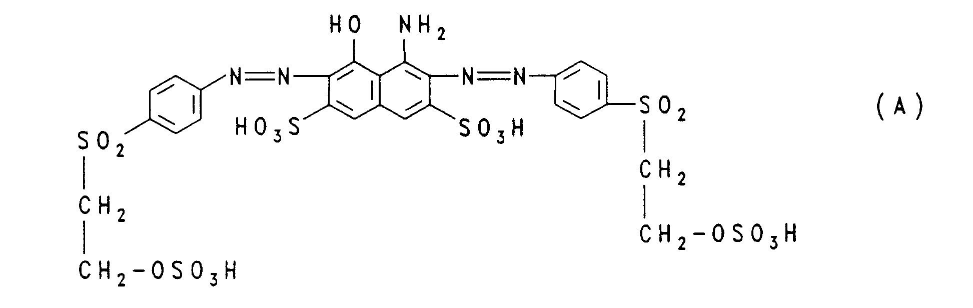

12 Teile des goldgelbfärbenden Monoazofarbstoffes der Formel (B) 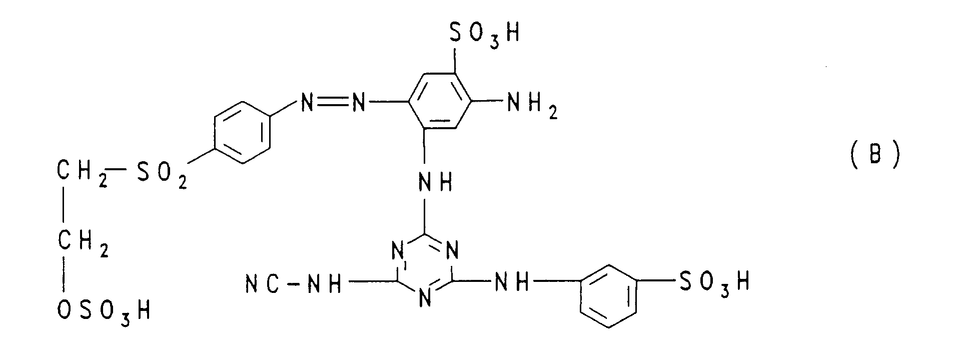
und 6 Teile des roten Monoazofarbstoffes der Formel (C) 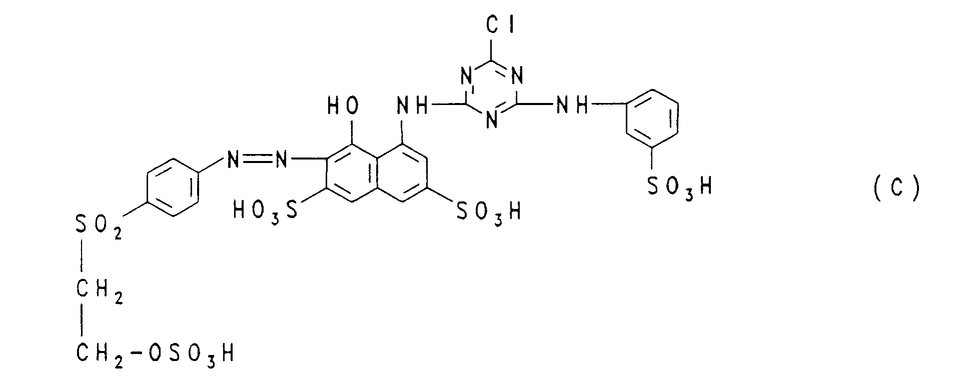
werden in einem mechanischen Mischer miteinander gemischt. Die erhaltene erfindungsgemäße Farbstoffmischung ergibt nach den in der Technik für faserreaktive Farbstoffe üblichen Applikations- und Fixierverfahren beispielsweise auf Cellulosefasermaterialien Färbungen und Drucke in tiefen schwarzen Tönen.

### Beispiel 2

22 Teile des marineblaufärbenden Disazofarbstoffes der Formel (A), 14 Teile des goldgelbfärbenden Monoazofarbstoffes der Formel (D) 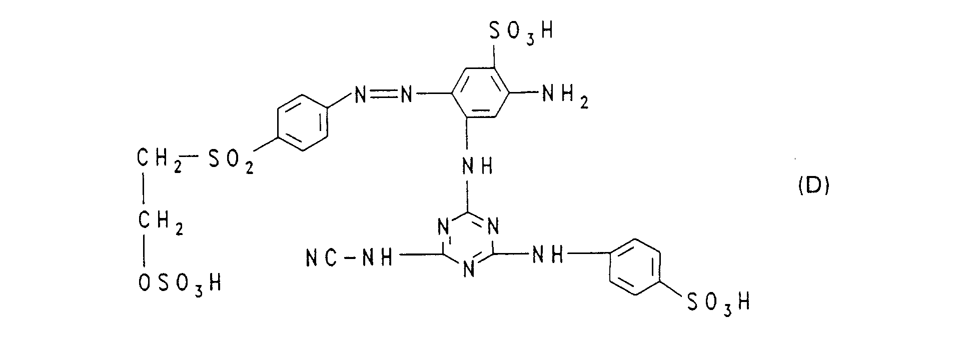
und 6 Teile des roten Monoazofarbstoffes der Formel (C) werden in einem mechanischen Mischer miteinander gemischt. Die erhaltene erfindungsgemäße Farbstoffmischung ergibt nach den in der Technik für faserreaktive Farbstoffe üblichen Applikations- und Fixierverfahren beispielsweise auf Cellulosefasermaterialien Färbungen und Drucke in tiefen schwarzen Tönen.

### Beispiel 3

27 Teile des marineblaufärbenden Disazofarbstoffes der Formel (A), 10 Teile des goldgelbfärbenden Monoazofarbstoffes der Formel (B) und 4,8 Teile des roten Monoazofarbstoffes der Formel (E) 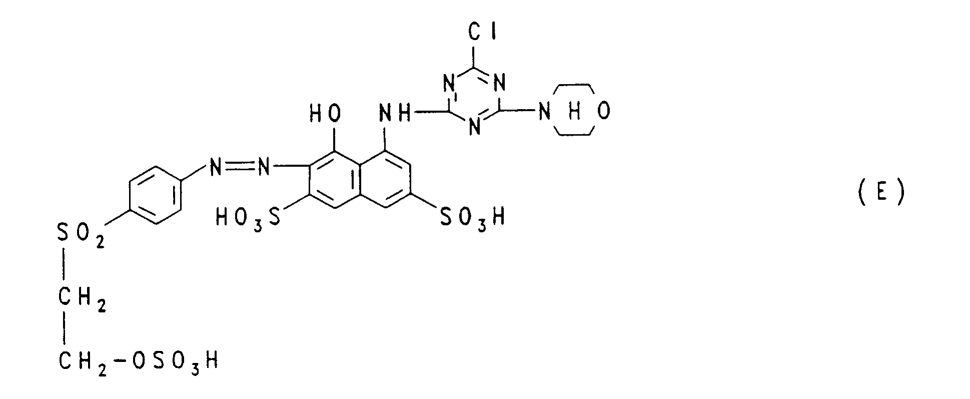
werden in einem mechanischen Mischer miteinander gemischt. Die erhaltene erfindungsgemäße Farbstoffmischung ergibt nach den in der Technik für faserreaktive Farbstoffe üblichen Applikations- und Fixierverfahren beispielsweise auf Cellulosefasermaterialien Färbungen und Drucke in tiefen schwarzen Tönen.

### Beispiel 4

24,8 Teile des marineblaufärbenden Disazofarbstoffes der Formel (A), 12 Teile des goldgelbfärbenden Monoazofarbstoffes der Formel (B) und 4,8 Teile des roten Disazofarbstoffes der Formel (F) 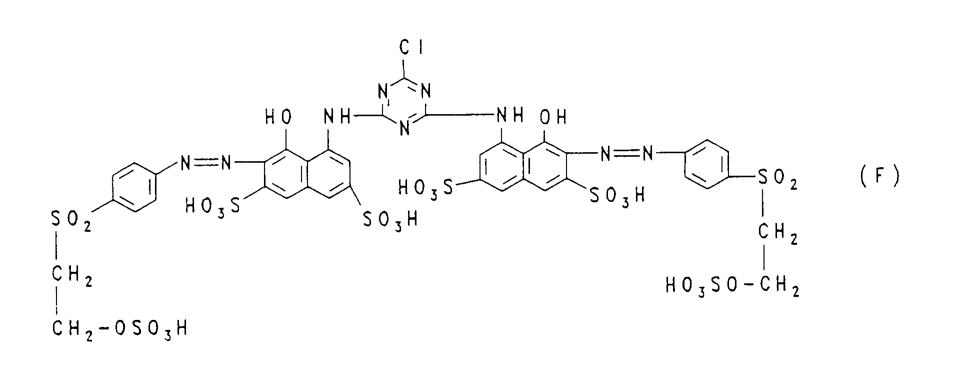
werden in einem mechanischen Mischer miteinander gemischt. Die erhaltene erfindungsgemäße Farbstoffmischung ergibt nach den in der Technik für faserreaktive Farbstoffe üblichen Applikations- und Fixierverfahren beispielsweise auf Cellulosefasermaterialien Färbungen und Drucke in tiefen schwarzen Tönen.

### Beispiel 5

24,8 Teile des marineblaufärbenden Disazofarbstoffes der Formel (A), 12 Teile des goldgelbfärbenden Monoazofarbstoffes der Formel (B) und 6 Teile des roten Monoazofarbstoffes der Formel (G) 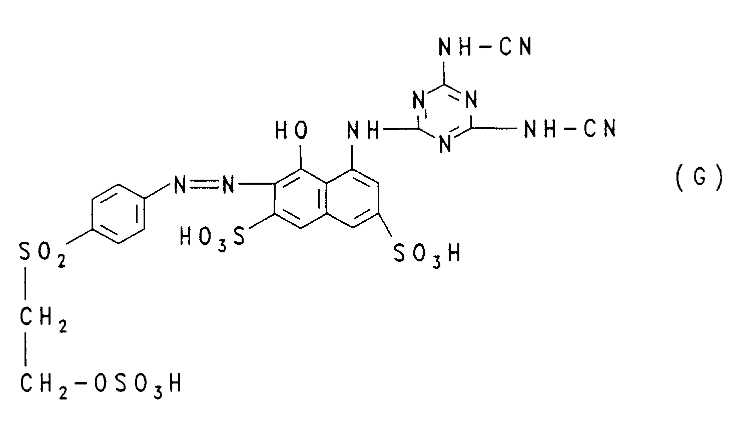
werden in einem mechanischen Mischer miteinander gemischt. Die erhaltene erfindungsgemäße Farbstoffmischung ergibt nach den in der Technik für faserreaktive Farbstoffe üblichen Applikations- und Fixierverfahren beispielsweise auf Cellulosefasermaterialien Färbungen und Drucke in tiefen schwarzen Tönen.

### Beispiel 6

24,8 Teile des marineblaufärbenden Disazofarbstoffes der Formel (A), 12 Teile des goldgelbfärbenden Monoazofarbstoffes der Formel (H) 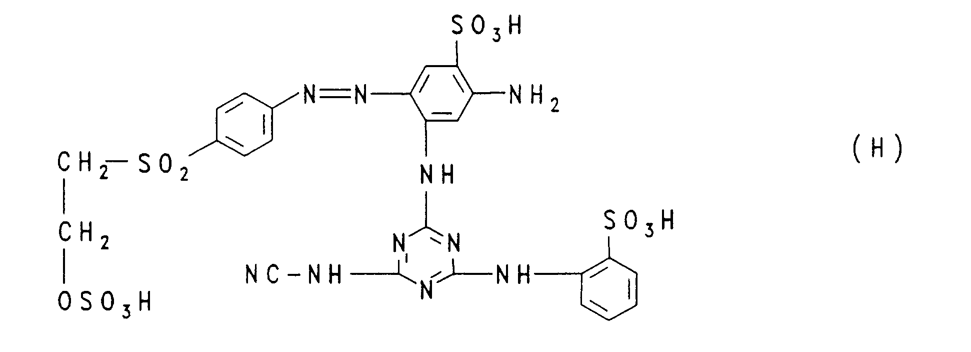
und 6 Teile des roten Monoazofarbstoffes der Formel (C) werden in einem mechanischen Mischer miteinander gemischt. Die erhaltene erfindungsgemäße Farbstoffmischung ergibt nach den in der Technik für faserreaktive Farbstoffe üblichen Applikations- und Fixierverfahren beispielsweise auf Cellulosefasermaterialien Färbungen und Drucke in tiefen schwarzen Tönen.

### Beispiel 7

24,8 Teile des marineblaufärbenden Disazofarbstoffes der Formel (A), 12 Teile des goldgelbfärbenden Monoazofarbstoffes der Formel (H) und 6 Teile des roten Monoazofarbstoffes der Formel (E) werden in einem mechanischen Mischer miteinander gemischt. Die erhaltene erfindungsgemäße Farbstoffmischung ergibt nach den in der Technik für faserreaktive Farbstoffe üblichen Applikations- und Fixierverfahren beispielsweise auf Cellulosefasermaterialien Färbungen und Drucke in tiefen schwarzen Tönen.

### Beispiel 8

24,8 Teile des marineblaufärbenden Disazofarbstoffes der Formel (A), 12 Teile des goldgelbfärbenden Monoazofarbstoffes der Formel (J) 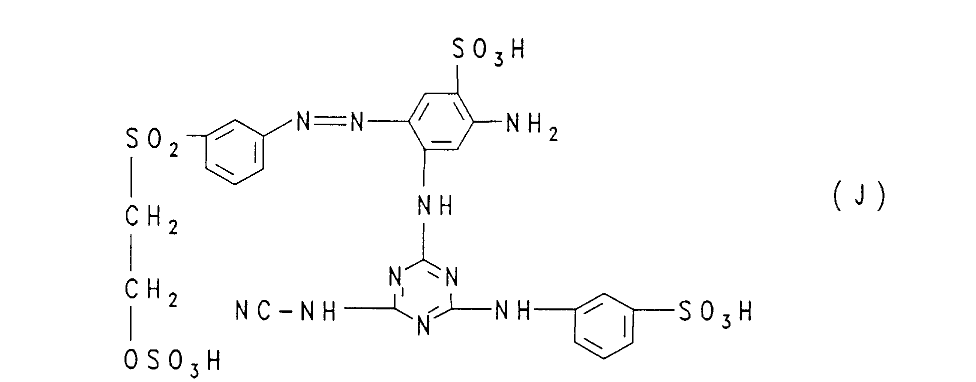
und 6 Teile des roten Monoazofarbstoffes der Formel (C) werden in einem mechanischen Mischer miteinander gemischt. Die erhaltene erfindungsgemäße Farbstoffmischung ergibt nach den in der Technik für faserreaktive Farbstoffe üblichen Applikations- und Fixierverfahren beispielsweise auf Cellulosefasermaterialien Färbungen und Drucke in tiefen schwarzen Tönen.

### Beispiel 9

442 Teile 4-(ß-Sulfatoethylsulfonyl)-anilin werden in bekannter Weise diazotiert. Zu der sauren Diazoniumsalzsuspension werden bei 10 bis 150 C 176 Teile 1-Amino-8-naphthol-3,6-disulfonsäure hinzugegeben und die Kupplungsreaktion unter Einhaltung eines pH-Wertes zwischen 1 und 2 durchgeführt. Nach etwa 8 bis 10 Stunden stellt man mittels Natriumcarbonat einen pH-Wert von 12,5 ein und gibt 143 Teile der Verbindung 2-Cyanoamino-4-(3'-sulfophenyl)-amino-6-(4"-sulfo-3"-amino-phenyl)-amino-1,3,5-triazin, gelöst in 600 Teilen Eiswasser, sowie 78 Teile der Verbindung 1-[2'-Chlor-4'-(3"-sulfophenyl)-amino-l',3',5'-triazin-6'-yl]-amino-8-hydroxy-naphthalin-3,6-disulfonsäurehinzu. Man rührt den Ansatz noch etwa 2 bis 3 Stunden bei 10°C unter Einhaltung eines pH-Wertes zwischen 5 und 6 nach und isoliert sodann das erfindungsgemäße Farbstoffgemisch, enthaltend etwa 500 Teile des Farbstoffes der Formel (A), 220 Teile des Farbstoffes der Formel (B) und 130 Teile des Farbstoffes (C), aus der Syntheselösung durch Sprühtrocknung. Es zeigt dieselben guten anwendungstechnischen Eigenschaften wie die Farbstoffmischung von Beispiel 1 und liefert beispielsweise auf Cellulosefasermaterialien tiefe schwarze Färbungen und Drucke.

### Beispiel 10

Zur Herstellung eines erfindungsgemäßen Farbstoffgemisches verfährt man gemäß der Verfahrensweise des Beispieles 9 unter Einsatz des Diazoniumsalzes aus 442 Teilen 4-(ß-Sulfatoethylsulfonyl)-anilin und, als Kupplungskomponenten, von 176 Teilen 1-Amino-8-naphthol-3,6-disulfonsäure,143 Teilen 2-Cyanoamino-4-(3'-sulfophenyl)-amino-6-(4"-sulfo-3"-amino-phenyl)-amino-1,3,5-triazin und 75 Teilen 1-(2',4'-Di-cyanoamino-1',3',5'-triazin-6'-yl)-amino-8-hydroxy-naphthalin-3,6-disulfonsäure. Die erfindungsgemäße Farbstoffmischung, enthaltend 500 Teile des Farbstoffes der Formel (A), 220 Teile des Farbstoffes der Formel (B) und 120 Teile des Farbstoffes der Formel (G), wird aus der Syntheselösung durch Sprühtrocknung isoliert. Sie zeigt dieselben guten anwendungstechnischen Eigenschaften wie die Farbstoffmischung von Beispiel 1 und liefert beispielsweise auf Cellulosefasermaterialien tiefe schwarze Färbungen und Drucke.

### Beispiel 11

Zur Herstellung eines erfindungsgemäßen Farbstoffgemisches verfährt man gemäß der Verfahrensweise des Beispieles 9 unter Einsatz des Diazoniumsalzes aus 442 Teilen 4-(ß-Sulfatoethylsulfonyl)-anilin und, als Kupplungskomponenten, von 176 Teilen 1-Amino-8-naphthol-3,6-disulfonsäure,143 Teilen 2-Cyanoamino-4-(3'-sulfophenyl)-amino-6-(4"sulfo-3"-amino-phenyl)-amino-1,3,5-triazin und 60 Teile 1-(2'-Fluor-4'-morpholino-1',3',5'-triazin-6'-yl)-amino-8-hydroxy-naphthalin-3,6-disulfonsäure. Die erfindungsgemäße Farbstoffmischung, enthaltend 550 Teile des Farbstoffes der Formel (A), 220 Teile des Farbstoffes der Formel (B) und 95 Teile des Farbstoffes der Formel (E), wird aus der Syntheselösung durch Sprühtrocknung isoliert. Sie zeigt dieselben guten anwendungstechnischen Eigenschaften wie die Farbstoffmischung von Beispiel 1 und liefert beispielsweise auf Cellulosefasermaterialien tiefe schwarze Färbungen und Drucke.

### Beispiel 12

Zur Herstellung eines erfindungsgemäßen Farbstoffgemisches verfährt man gemäß der Verfahrensweise des Beispieles 9 unter Einsatz des Diazoniumsalzes aus 442 Teilen 4-(ß-Sulfatoethylsulfonyl)-anilin und, als Kupplungskomponenten, von 176 Teilen 1-Amino-8-naphthol-3,6-disulfonsäure,143 Teilen 2-Cyanoamino-4-(3'-sulfophenyl)-amino-6-(4"-sulfo-3"-amino-phenyl)-amino-1,3,5-triazin und 45 Teilen 2-Chlor-4,6-di-(3',6'-disulfo-8'-hydroxy-naphth-1'-yl-amino)-1,3,5-triazin. Die erfindungsgemäße Farbstoffmischung, enthaltend 550 Teile des Farbstoffes der Formel (A), 220 Teile des Farbstoffes der Formel (B) und 80 Teile des Farbstoffes der Formel (F), wird aus der Syntheselösung durch Sprühtrocknung isoliert. Sie zeigt dieselben guten anwendungstechnischen Eigenschaften wie die Farbstoffmischung von Beispiel 1 und liefert beispielsweise auf Cellulosefasermaterialien tiefe schwarze Färbungen und Drucke.

### Beispiel 13

Zur Herstellung eines erfindungsgemäßen Farbstoffgemisches verfährt man gemäß der Verfahrensweise des Beispieles 9 unter Einsatz des Diazoniumsalzes aus 442 Teilen 4-(ß-Sulfatoethylsulfonyl)-anilin und, als Kupplungskomponenten, von 176 Teilen 1-Amino-8-naphthol-3,6-disulfonsäure,143 Teilen 2-Cyanoamino-4-(4'-sulfophenyl)-amino-6-(4"-sulfo-3"-amino-phenyl)-amino-1,3,5-triazin und 78 Teile 1-[2'-Chlor-4'-(3"- sulfophenyl)-amino-1',3',5'-triazin-6'-yl]-amino-8-hydroxy-naphthalin-3,6-disulfonsäure. Die erfindungsgemäße Farbstoffmischung, enthaltend 550 Teile des Farbstoffes der Formel (A), 220 Teile des Farbstoffes der Formel (D) und 130 Teile des Farbstoffes der Formel (C), wird aus der Syntheselösung durch Sprühtrocknung isoliert. Es zeigt dieselben guten anwendungstechnischen Eigenschaften wie die Farbstoffmischung von Beispiel 1 und liefert beispielsweise auf Cellulosefasermaterialien tiefe schwarze Färbungen und Drucke.

### Beispiel 14

Zur Herstellung eines erfindungsgemäßen Farbstoffgemisches verfährt man gemäß der Verfahrensweise des Beispieles 9 unter Einsatz des Diazoniumsalzes aus 442 Teilen 4-(ß-Sulfatoethylsulfonyl)-anilin und, als Kupplungskomponenten, von 176 Teilen 1-Amino-8-naphthol-3,6-disulfonsäure,143 Teilen 2-Cyanoamino-4-(2'-sulfophenyl)-amino-6-(4"-sulfo-3"-amino-phenyl)-amino-1,3,5-triazin und 78 Teilen 1-[2'-Chlor-4'-(3"-sulfophenyl)-amino-1',3',5'-triazin-6'-yl]-amino-8-hydroxy-naphthalin-3,6-disulfonsäure. Die erfindungsgemäße Farbstoffmischung, enthaltend 550 Teile des Farbstoffes der Formel (A), 220 Teile des Farbstoffes der Formel (H) und 130 Teile des Farbstoffes der Formel (C), wird aus der Syntheselösung durch Sprühtrocknung isoliert. Es zeigt dieselben guten anwendungstechnischen Eigenschaften wie die Farbstoffmischung von Beispiel 1 und liefert beispielsweise auf Cellulosefasermaterialien tiefe schwarze Färbungen und Drucke.

### Beispiel 15

Die Farbstoffe der Formeln (A), (B) und (C) werden in üblicher Weise in getrennten Ansätzen synthetisiert. 400 Vol.-Teile der wäßrigen Syntheselösung mit 25 Teilen des Farbstoffes (A), 200 Vol.-Teilen der wäßrigen Syntheselösung mit 12 Teilen des Farbstoffes (B) und 80 Vol.-Teile der wäßrigen Syntheselösung mit 6 Teilen des Farbstoffes (C) werden miteinander vermischt und die Farbstoffmischung durch Sprühtrocknung isoliert. Man erhält die Farbstoffmischung des Beispieles 1 mit den gleichen guten färberischen Eigenschaften.

### Beispiel 16

24,8 Teile des marineblaufärbenden Disazofarbstoffes der Formel (A) und 16 Teile des goldgelbfärbenden Monoazofarbstoffes der Formel (B) werden in einem mechanischen Mischer miteinander homogen vermischt. Die erhaltene erfindungsgemäße Farbstoffmischung ergibt nach den in der Technik für faserreaktive Farbstoffe üblichen Anwendungsverfahren beispielsweise auf Cellulosefasermaterialien Färbungen und Drucke in tiefen grünstichig schwarzen Tönen.

## Patentansprüche

1. Farbstoffmischung, enthaltend einen oder mehrere Disazofarbstoffe entsprechend der allgemeinen Formel (1) und einen oder mehrere Mono- oder Disazofarbstoffe entsprechend der allgemeinen Formel (2A) oder (2B) oder eine Mischung dieser Azofarbstoffe der Formeln (2) 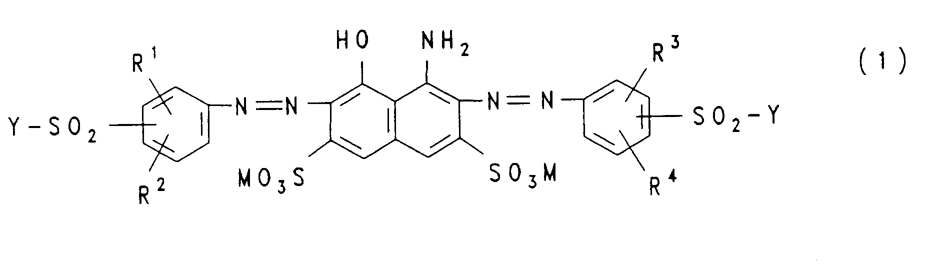 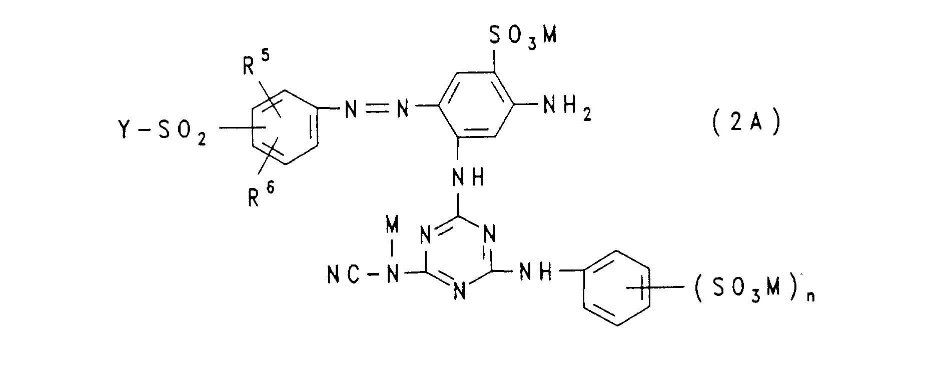 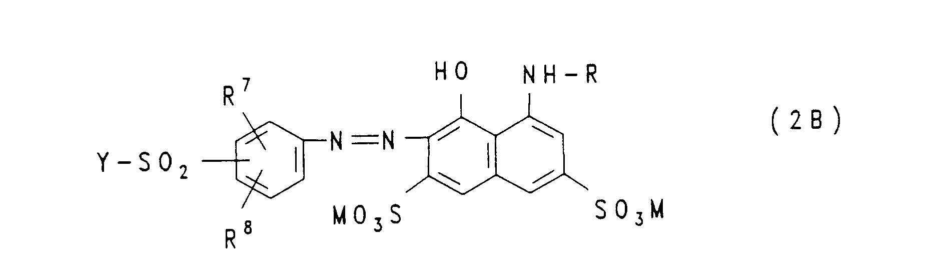
worin bedeuten:
M ist Wasserstoff oder ein Alkalimetall;
R¹ ist Wasserstoff, Methyl, Ethyl, Methoxy, Ethoxy, Sulfo oder Carboxy;
R² ist Wasserstoff, Methyl, Ethyl, Methoxy oder Ethoxy;
R³ ist Wasserstoff, Methyl, Ethyl, Methoxy, Ethoxy, Sulfo oder Carboxy;
R⁴ ist Wasserstoff, Methyl, Ethyl, Methoxy oder Ethoxy;
R⁵ ist Wasserstoff, Methyl, Ethyl, Methoxy, Ethoxy, Sulfo oder Carboxy;
R⁶ ist Wasserstoff, Methyl, Ethyl, Methoxy oder Ethoxy;
R⁷ ist Wasserstoff, Methyl, Ethyl, Methoxy, Ethoxy, Sulfo oder Carboxy;
R⁸ ist Wasserstoff, Methyl, Ethyl, Methoxy oder Ethoxy;
n ist die Zahl 1 oder 2;
Y ist jedes, unabhängig voneinander, Vinyl, ß-Chlorethyl, β-Thiosulfatoethyl oder β-Sulfatoethyl;
R ist Alkanoyl von 2 bis 5 C-Atomen, Benzoyl oder 2,4-Di-(cyanoamino)-1,3,5-triazin-6-yl oder eine Gruppe der allgemeinen Formel (3a) oder (3b) 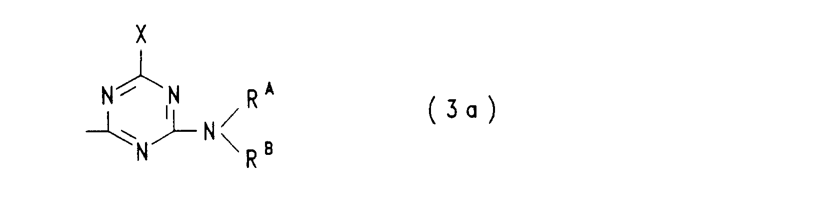 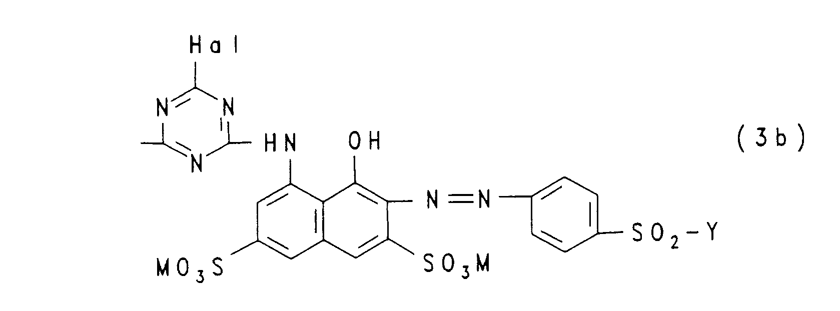
in welchen
M und Y eine der obengenannten Bedeutungen haben,
X Chlor, Fluor oder Cyanoamino ist,
Hal Chlor oder Fluor ist,
R^{A} Wasserstoff, Alkyl von 1 bis 4 C-Atomen, Phenyl oder substituiertes Phenyl ist und
R Monosulfo-phenyl oder Disulfo-phenyl ist oder
R^{A} und R zusammen mit dem N-Atom einen gesättigten heterocyclischen Rest mit einem Alkylen von 4 bis 7 C-Atomen oder mit 2 Alkylenen von 1 bis 4 C-Atomen und einer weiteren Heterogruppe bilden.

2. Farbstoffmischung nach Anspruch 1, dadurch gekennzeichnet, daß die Farbstoffe der allgemeinen Formel (1) und die Farbstoffe der allgemeinen Formeln (2) in einem molaren Mischungsverhältnis von 1:0,3 bis 1:1,25 in der Mischung enthalten sind.

3. Farbstoffmischung nach Anspruch 1, dadurch gekennzeichnet, daß die Farbstoffe der allgemeinen Formel (1) und die Farbstoffe der allgemeinen Formeln (2) in einem molaren Mischungsverhältnis von 1:0,4 bis 1:0,8 in der Mischung enthalten sind.

4. Farbstoffmischung nach mindestens einem der Ansprüche 1 bis 3, dadurch gekennzeichnet, daß in der Mischung sowohl ein oder mehrere der Farbstoffe der allgemeinen Formel (2A) und ein oder mehrere der Farbstoffe der allgemeinen Formel (2B) enthalten sind.

5. Farbstoffmischung nach Anspruch 4, dadurch gekennzeichnet, daß das molare Mischungsverhältnis zwischen den Farbstoffen (2A) und (2B) 1:0,2 bis 1:1 beträgt.

6. Farbstoffmischung nach mindestens einem der Ansprüche 1 bis 5, dadurch gekennzeichnet, daß R¹, R³, R⁵ und R⁷ jedes, unabhängig voneinander, Wasserstoff oder Methoxy ist und R², R⁴, R⁶ und R⁸ jedes Wasserstoff ist.

7. Farbstoffmischung nach mindestens einem der Ansprüche 1 bis 5, dadurch gekennzeichnet, daß die Substituenten R¹ bis R⁸ jedes Wasserstoff bedeuten.

8. Farbstoffmischung nach mindestens einem der Ansprüche 1 bis 7, dadurch gekennzeichnet, daß R ein Rest der in Anspruch 1 genannten und definierten allgemeinen Formel (3a) oder (3b) ist.

9. Farbstoffmischung nach mindestens einem der Ansprüche 1 bis 7, dadurch gekennzeichnet daß R ein Rest der in Anspruch 1 genannten und definierten allgemeinen Formel (3a) ist, in welcher X Chlor oder Fluor bedeutet.

10. Farbstoffmischung nach Anspruch 9, dadurch gekennzeichnet, daß R^ Wasserstoff ist und R^{B} 3-Sulfo-phenyl bedeutet.

11. Wäßrige Flüssigpräparation, enthaltend eine Farbstoffmischung nach mindestens einem der Ansprüche 1 bis 10 mit einem Gesamtfarbstoffgehalt von 5 bis 50 Gew.-%.

12. Verwendung einer Farbstoffmischung nach mindestens einem der Ansprüche 1 bis 11 oder der Farbstoffe (1) und (2) gemeinsam zum Färben von hydroxy- und/oder carbonamidgruppenhaltigem Fasermaterial.

13. Verfahren zum Färben von hydroxy- und/oder carbonamidgruppenhaltigem Fasermaterial, bei welchem man Farbstoffe in gelöster Form auf das Material aufbringt und diese auf dem Material mittels Wärme und/oder mit Hilfe eines alkalisch wirkenden Mittels fixiert, dadurch gekennzeichnet daß als Farbstoffe eine Farbstoffmischung von mindestens einem der Ansprüche 1 bis 11 oder die Farbstoffe (1) und (2) gemeinsam in der Färbelösung einsetzt.
